# EUROPEAN PATENT APPLICATION

(11) **EP 1 604 686 A1**
(43) Date of publication of application: **14.12.2005**
(21) Application number: 05076326.7
(22) Date of filing: 08.06.2005
(51) Int. Cl.: A61K 45/06, A61K 31/407, A61K 31/366, A61P 25/28

(54) **Use of phenserine and a HMG CoA reductase inhibitor for delaying Alzheimer's disease progression**

(30) Priority: 08.06.2004 US 577959 P
(71) Applicant: Axonyx, Inc., New York, NY 10018 (US)
(72) Inventor: Bruinsma, Gosse B., 2312 TT Leiden (NL)
(74) Representative: Winckels, Johannes Hubertus F.

(57) **Abstract**

The present invention relates to compositions and methods of treating a cognitive disorder in a subject by administering an effective amount of a phenserine compound or a pharmaceutically acceptable salt thereof and an effective amount of a HMG CoA reductase inhibitor or a pharmaceutically acceptable salt thereof to a subject, thereby treating a cognitive disorder in the subject.

## Description

### TECHNICAL FIELD

This invention relates to pharmaceutical compositions and methods of using a β-amyloid precursor protein inhibitor (β-APP), such as a phenserine compound, in combination with a HMG CoA reductase inhibitor.

### BACKGROUND

Alzheimer's disease ("AD") is a form of dementia in which subjects develop progressive neurodegeneration, complete loss of cognitive abilities, and die prematurely. Alzheimer's disease is a complex cognitive disorder associated with major structural changes in the brain; loss of neurons in the hippocampus and cortex, accumulation of intracellular protein deposits (neurofibrillary tangles) and accumulation of extracellular protein deposits (amyloid or senile plaques). The major component of the senile plaques is amyloid β-peptide (Aβ) which resides within a much larger amyloid precursor protein (β-APP). Normal metabolism of β-APP involves proteolytic cleavage within the Aβ region which precludes the formation of amyloidogenic Aβ peptides, and favors the release of non-amyloidogenic soluble β-APP fragments into the extracellular fluid. Aβ formation can increase due to β-APP overexpression or missense mutations which alter constitutive β-APP processing pathways. Evidence suggests that although non-amyloidogenic β-APPs are neuroprotective, Aβ may be one of the main causes for cytotoxic processes leading to neuronal death in Alzheimer's disease.

In addition, defects in the cholinergic system have been suggested to underlie cognitive impairments associated with normal aging and Alzheimer's disease (Bartus et al., Science 217:408-417 (1982); Fisher et al., Neurobiol. Aging 13:9-23 (1992)). Much research has focused on the development of cholinomemetic replacement therapy as a potential treatment of these impairments. Among them, cholinesterase inhibitors, such as physostigmine ("Phy") and tetrahydroaminoacridine ("ThA") have been investigated for memory-enhancing effects in both animals (Rupniak et al., Neurobiol. Aging 11:09-613; 1990); Murray et al., Psychopharmacology 105:134-136 (1991)) and human patients (Mohs et al., J. Am. Geriatr. Soc. 3:749-757 (1985); Summers et al., N. Engl. J. Med. 315:1241-1245(1986)).

Phenserine compounds of the invention reduce the production of β-APP and Aβ. Phenserine ((-)-N-phenylcarbamoyl eseroline) has also been identified as a selective AChE inhibitor ("AChEI") and thus suited as an agent for the therapy for cognitive impairments associated with aging and Alzheimer's disease (for example, *see,* U.S. Patents 5,409,948, issued April 25, 1995, to Greig *et al*.; and 5,171,750, issued December 15, 1992, to Brossi *et al*., the contents of both of which are incorporated by reference). Hence, acetylcholinesterase AChE, which degrades ACh, is an additional target in the treatment of Alzheimer's Disease.

At the present time Alzheimer's disease cannot be cured. However, strategies that reduce β-APPs production, prevent the formation of Aβ, or reduce Aβ toxicity, would appear to retard the progression of Alzheimer's disease, thereby providing a beneficial treatment. In addition, cognitive functions may be improved through the administration of an AChE inhibitor (AChEI).

### DISCLOSURE OF THE INVENTION

The invention relates to a method of treating or delaying a cognitive disorder in a subject, comprising administering an effective amount of a phenserine compound or a pharmaceutically acceptable salt or ester thereof to a subject and administering an effective amount of a HMG CoA reductase inhibitor (i.e., a statin) or a pharmaceutically acceptable salt thereof to the subject, thereby treating the cognitive disorder. In an exemplary embodiment, the invention provides a method of treating AD by reducing production of Aβ by administering a phenserine compound that reduces the production of Aβ (an Aβ inhibitor, AβI), for example, by decreasing translation of a β-APP encoding mRNA, and a HMG CoA reductase inhibitor to reduce an inflammatory response in a subject, which inflammatory response may be measured by the presence of markers of inflammation such as the C-reactive protein.

The present invention also provides a method of treating a cognitive disorder by administering an effective amount of a statin selected from the group consisting of atorvastatin, rivastatin, mevastatin, lovastatin, pravastatin, velostatin, fluvastatin, and a combination thereof. In another exemplary embodiment, the statin may be simvastatin.

The present invention further relates to a method of treating and a composition useful in the treatment of cognitive disorders, for example, AD, dementia, age related dementia, vascular dementia, and/or Parkinson's disease. In an exemplary embodiment, an AβI and a HMG CoA reductase inhibitor are coadministered to a subject to improve cognitive function and/or retard progression and/or onset of the cognitive disorder, for example, AD.

The present invention further relates to a method of treating and a composition useful in the treatment of cognitive disorders, for example, AD, dementia, age related dementia, vascular dementia, and/or Parkinson's disease. In an exemplary embodiment, an AβI and a HMG CoA reductase inhibitor are coadministered to a subject in conjunction with an additional compound, such as a second phenserine compound, to improve cognitive function and/or retard progression and/or onset of the cognitive disorder, for example, AD. Optionally, the second phenserine compound may be (+) 9 - N- phenylcarbinol esroline ((+)-phenserine; or POSIPHEN™).

The present invention also relates to a method of manufacturing a pharmaceutical composition comprising a phenserine compound or a pharmaceutically acceptable salt or ester thereof and a HMG CoA reductase inhibitor or a pharmaceutically acceptable salt or ester thereof for the treatment or prevention of a disorder associated with the production of beta-amyloid protein (Aβ), for example, AD, age related dementia, dementia, vascular dementia, and/or Parkinson's disease.

Optionally, the methods and compositions of the invention may be administered to a subject that does not contain a mutation in the APOE 4 gene. Optionally, the HMG-CoA reductase inhibitor is not simvastatin.

### MODES FOR CARRYING OUT THE INVENTION

AD is a degenerative condition affecting memory, judgment and the ability to reason that affects about 4.5 million Americans. Provided herein is an improved method of treating cognitive diseases, such as AD, dementia, vascular dementia, and Parkinson's disease.

Drugs such as phenserine are thought to act by increasing the availability of the neurotransmitter acetylcholine. In addition, phenserine compounds of the invention are inhibitors of Aβ production. In contrast, HMG CoA reductase inhibitors are typically used in the treatment or prevention of coronary disease. The present invention provides a beneficial use of a HMG CoA reductase inhibitor in combination with a phenserine compound to produce an improved treatment of, for example, AD. For example, co-administration of a HMG CoA reductase inhibitor, which can reduce total serum cholesterol levels, and a phenserine compound, which reduces production of β-APP, provides a treatment that may help prevent the onset and/or slow the progression of AD. The administration of both compounds to a subject is believed to slow progression of the disease by at least slowing the accumulation of Aβ and reducing inflammation associated with the disease. In addition, the phenserine compounds of the invention may also provide additional neurotransmitter availability, thereby boosting the cognitive ability of a subject as an additional benefit of the treatment.

As used herein, the phrases "*co-administration*," "*in combination with,*" "*the combination of*" or similar phrases referring to two or more drugs or compounds means that the compounds are present in the subject being treated at the same time. The compounds may be administered at the same time or sequentially in any order at different points in time. However, the compounds should be administered sufficiently closely in time so as to provide the desired enhancement of treatment effect. The compounds may be administered by the same route of administration or by different routes of administration. Suitable dosing intervals, routes and the order of administration with such compounds will be readily apparent to those skilled in the art, in light of the present disclosure.

As used herein, "*effective amount*" means an amount of an active ingredient administered to the subject, which will be effective to delay onset of or treat the disease condition in the subject.

HMG CoA reductase is the enzyme that catalyzes an early step in the cholesterol biosynthesis pathway. Specifically, the enzyme converts HMG-CoA to mevalonate. Cholesterol and triglycerides, which circulate in the bloodstream as part of lipoprotein complexes, can be separated by density ultracentrifugation into high (HDL), intermediate (IDL), low (LDL), and very low (VLDL) density lipoprotein fractions. Triglycerides (TG) and cholesterol synthesized in the liver are incorporated into VLDLs and released into the plasma for delivery to pheripheral tissues. In a series of subsequent steps, VLDLs are transformed into IDLs and cholesterol-rich LDLs. HDLs, containing apolipoprotein A, are hypothesized to participate in the reverse transport of cholesterol from tissues back to the liver.

Clinical and pathological studies have shown that elevated levels of total cholesterol, low LDL-cholesterol (LDL-C), and apolipoprotein B (a membrane transport protein for LDL) promote human atherosclerosis. Similarly, decreased levels of HDL-cholesterol (HDL-C) and its transport complex, apolipoprotein A, are associated with the development of atherosclerosis. Epidemiologic investigations have established that cardiovascular morbidity and mortality vary directly with the level of total cholesterol and LDL-C, and inversely with the level of HDL-C. Thus, HDLs have been characterized as "good" lipoproteins, while cholesterol-rich LDLs have been characterized as being less favorable.

Elevated serum total cholesterol is closely related to the development of cardiovascular, cerebrovascular, and peripheral vascular disorders. Hypercholesterolemia has been linked to increased risk of coronary heart disease. Many studies have found that a reduction of elevated serum cholesterol levels leads to a decreased incidence of coronary disease.

HMG CoA reductase inhibitors have also been implicated in the reduction of inflammatory responses, however, such reports are believed to be inconclusive. Furthermore, the relationship between inflammation and AD remains controversial. Hence, a person of ordinary skill in the art has no motivation to combine a HMG-CoA reductase inhibitor having an anti-inflammatory activity with a phenserine compound that reduces Aβ production, particularly where such a combination produces a synergistic effect.

The HMG CoA reductase inhibitors suitable for use in the invention include, but are not limited to, pravastatin *(see,* U.S. Patent 4,346,227, issued August 24, 1982, to Terahara *et al*.); lovastatin (*see*, U.S. Patent 4,231,938, issued November 4, 1980, to Monaghan *et al*.); velostatin, atorvastatin (LIPITOR®) (*see*, U.S. Patent 4,647,576, issued March 3, 1987, to Hoefle *et al*.); fluvastatin (LESCOL®) *(see,* U.S. Patents 5,354,772, issued October 11, 1994, to Kathawala; and 5,356,896, issued October 18, 1994, to Kabadi *et al*.); fluindostatin (Sandoz XU-62-320); pyrazole analogs of mevalonolactone derivatives (*see,* International publication WO 86/03488); rosuvastatin *(see,* U.S. Patents 6,589,959, issued July 8, 2003, to Taylor; 6,316,460, issued November 13, 2001, to Creekmore *et al*.; and RE37,314, issued August 7, 2001, to Hirai *et al*.);and rivastatin and other pyridyldihydroxyheptenoic acids (*see,* European Patent 491226A, published August 14, 1996, to Angerbauer *et al*.), as well as related compounds (*see also*, HMG CoA REDUCTASE INHIBITOR in THERAPEUTIC CATEGORY AND BIOLOGICAL ACTIVITY INDEX of THE MERK INDEX (13^{th} edt. 2001).

Methods of manufacturing and administrating statin's for treatment of coronary diseases are well known in the art. An effective dosage of a statin is preferably determined by a physician based on the factors such as the age, weight and medical condition of a subject. Statins have exhibited an acceptable safety and tolerability profile in the reduction of cholesterol levels.

As used herein, "*a phenserine compound*" means a compound structurally related to phenserine (*i.e.,* (3aS)-1,3a,8-trimethyl-1,2,3,3a,8,8a-hexahydropyrrolo[2,3-b]indol-5-yl phenylcarbamate or (-)-phenserine), such as (+)-N¹,N⁸-bisnorcymserine, (-)-N¹,N⁸-bisnorcymserine, (+)-phenserine (*i*.*e*., POSIPHEN™ or (3aR)-1,3a,8-trimethyl-1,2,3,3a,8,8a-hexahydropyrrolo[2,3-b]indol-5-yl phenylcarbamate), (+)-tolserine, (-)-tolserine *(see,* International Publication WO 02/48150) and pharmaceutically acceptable salts and esters thereof that inhibit β-APP production. For the sake of brevity the compound name is used herein to describe the invention and any such reference includes within its meaning pharmaceutically acceptable salts and esters thereof. Hence, reference to the compound phenserine, includes pharmaceutically acceptable salts and esters thereof, for example, phenserine tartrate.

The invention also includes A kit for treatment or prevention of a cognitive disorder, the kit comprising a first dosage unit comprising an effective amount of a phenserine compound or a pharmaceutically acceptable salt or ester thereof and a second dosage unit comprising an effective amount of a HMG CoA reductase inhibitor or pharmaceutically acceptable salt or ester thereof. Optionally, the kit may include instructions for use and/or packaging containing an appropriate dosage for each day or period of time. Preferably the phenserine compound of the kit comprises (-)-phenserine, (-)-phenserine tartrate, (+)-phenserine, (+)-phenserine tartrate, N¹,N⁸-bisnorcymserine (either or both enantiomers), and tolserine (either or both enantiomers).

Phenserine, in addition to being an AβI, is also a highly selective AChEI that produces fewer undesirable side effects, compared to physostigmine and tacrine, and robustly enhances cognition in animal models *(see,* Greig NH *et al*. (2000) The Experimental Alzheimer Drug Phenserine: Preclinical Pharmacokinetics and Pharmacodynamics, *Acta Neurol Scand Suppl*. 176:74-84). To determine the time-dependent effects of phenserine on cholinergic function, AChE activity, brain and plasma drug levels and brain extracellular acetylcholine (ACh) concentrations were measured in rats before and after phenserine administration. Following i.v. dosing, brain drug levels were 10-fold higher than those achieved in plasma, peaked within 5 min and rapidly declined with half-lives of 8.5 and 12.6 min, respectively. In contrast, a high (> 70%) and long-lasting inhibition of AChE was achieved (half-life > 8.25 h). Striatal, *in vivo* microdialysis in conscious, freely-moving phenserine-treated rats demonstrated a greater than 3-fold rise in brain ACh levels. Phenserine, thus, is rapidly absorbed and cleared from the body, but produces a long-lasting stimulation of brain cholinergic function at well tolerated doses and hence has superior properties as a drug for cognitive disorders, such as AD. Thus, phenserine provides an additional benefit in the present invention by increasing acetylcholine levels in the subject.

The effective dose of phenserine, a representative example of a phenserine compound, in mammals, for example, a human, may vary due to such factors as age, weight, activity level or condition of the subject being treated. Typically, an effective dosage of a compound according to the present invention is about 1 to 800 milligrams when administrated by either oral or rectal dose from 1 to 3 times daily. This dosage is typically about 0.002 to about 50 milligrams per kilogram of the subject's weight administered per day. Preferably, from about 10 to about 300 milligrams are administered orally or rectally 1 to 3 times a day for an adult human. In an exemplary embodiment, the dosage is between 5 and 60 mg twice a day (bid), including 10 mg *bid*, 15 mg *bid*, 20 mg *bid*, 25 mg *bid*, 30 mg *bid*, and 35 mg *bid*. The required dose is considerably less when administered parenterally. Preferably, from about 0.01 to about 150 milligrams may be administered intramuscularly or transdermally, one or two times a day for an adult human.

A phenserine compound can be administered in any pharmaceutically acceptable amount, for example, in amounts ranging from 0.001 gram to about 1 gram per kilogram of body weight, wherein the amount of phenserine (e.g., phenserine fumerate) is expressed as the equivalent weight of phenserine tartrate. In an exemplary embodiment, the compound is administered in a dosage of 5 mg twice a day. In another exemplary embodiment, the compound is administered in a dosage of 7.5 mg twice a day. In yet another exemplary embodiment, the compound is administered in a dosage of 10 mg twice a day. In yet another exemplary embodiment, the compound is administered in a dosage of 15 mg twice a day. In yet another exemplary embodiment, the compound is administered in a dosage of 20 mg twice a day. In yet another exemplary embodiment, the compound is administered in a dosage of 25 mg twice a day. In yet another exemplary embodiment, the compound is administered in a dosage of 30 mg twice a day. Preferably, an oral formulation of a phenserine compound is not administered with food. In an exemplary embodiment, a phenserine compound is administered to a subject for greater than six months, more preferably, greater than one year. However, using the information presented herein, the determination of an effective amount of a phenserine compound is well within the skill of the ordinary practitioner in the art.

The oral administration/ingestion of a phenserine compound elicits a lesser response in a subject, as compared to an equal dosage administrated parenterally, due to metabolism of the drug during transit through the gastrointestinal tract and into the general circulation system. Thus, the metabolic breakdown of the active drug may be at least partially circumvented by administering the drug by an alternative route. Examples of such alternative routes include buccal or sublingual administration and parenteral administration. Drugs administered by these routes avoid gut-wall and hepatic metabolism, thereby producing increased bioavailability as compared to oral administration.

The compounds of the invention are generally used in pharmaceutical compositions (wt %) containing the active ingredient with a carrier or vehicle in the composition in an amount of about 0.1 to 99 wt % and preferably about 25-85 wt %. The compounds may be formulated for pharmaceutical use using methods known in the art. *See,* for example, *Remington's Pharmaceutical Sciences,* 18th Ed. (1990, Mack Publishing Co., Easton, Pa.). Accordingly, incorporation of the active compounds and a slow release matrix may be implemented.

Either fluid or solid unit dosage forms can be readily prepared for oral administration. For example, admixed with conventional ingredients such as dicalcium phosphate, magnesium aluminum silicate, magnesium stearate, calcium sulfate, starch, talc, lactose, acacia, methyl cellulose and functionally similar materials as pharmaceutical excipients or carriers. A sustained release formulation may optionally be used. In older or incoherent subjects, sustained release formulations may even be preferred. Capsules may be formulated by mixing the compound with a pharmaceutical diluent which is inert and inserting this mixture into a hard gelatin capsule having the appropriate size. If soft capsules are desired, a slurry of the compound with an acceptable vegetable, light petroleum or other inert oil can be encapsulated by forming into a gelatin capsule.

Suspensions, syrups and elixirs may be used for oral administration or fluid unit dosage forms. A fluid preparation including oil may be used for oil soluble forms. A vegetable oil such as corn oil, peanut oil or a flower oil, for example, together with flavoring agents, sweeteners and any preservatives produces an acceptable fluid preparation. A surfactant may be added to water to form a syrup for fluid unit dosages. Hydro-alcoholic pharmaceutical preparations may be used having an acceptable sweetener, such as sugar, saccharin or a biological sweetener and a flavoring agent in the form of an elixir.

Pharmaceutical compositions for parenteral and suppository administration can also be obtained using techniques standard in the art.

In an exemplary embodiment, the compounds of the invention are prepared as pharmaceutical agent suitable for oral administration. In another exemplary embodiment, the compounds of the invention are prepared in transdermal parenteral formulation, which is particularly useful in preventing or treating Aβ accumulating, cholinergic disorders such as AD. Accordingly, compositions suitable for administration to these areas are particularly included within the invention. The above parenteral solutions or suspensions may be administered transdermally with a skin patch. In addition, where appropriate and desirable they may be given by injection in an appropriate vehicle, such as sesame oil.

Pharmaceutical carriers acceptable for the purposes of this invention are the known art carriers that do not adversely affect the drug, the host, or the material comprising the drug delivery device. Suitable pharmaceutical carriers include sterile water, saline, dextrose, dextrose in water or saline, condensation products of castor oil and ethylene oxide combining about 30 to 35 moles of ethylene oxide per mole of castor oil, liquid acid, lower alkanols, oils such as corn oil, peanut oil, sesame oil and the like, with emulsifiers such as mono- or di-glyceride of a fatty acid; or a phosphatide, e.g., lecithin, and the like; glycols, polyalkylene glycols, aqueous media in the presence of a suspending agent, for example, sodium carboxymethyl cellulose, sodium alginate, poly(vinylpyrrolidone), and the like, alone or with suitable dispensing agents such as lecithin, polyoxyethylene stearate, and the like. The carrier may also contain adjutants such as preserving agents, stabilizing agents, wetting agents, emulsifying agents and the like together with the compounds of this invention.

Suitable salts of the compounds of the invention, such as acid addition salts, which may be prepared according to a conventional procedure, are known in the art and include the following acids: hydrochloric, hydrobromic, methanesulfonic, isothionic, sulfuric, phosphoric, and sulfamic acids and, from the organic series: acetic, propionic, maleic, fumaric, tartaric, citric, oxalic, and benzoic acids, to name a few. Memantine acids include, hydrochloric, citric, and maleic. Other pharmaceutically-acceptable acid addition salts may be prepared, if desired, and one acid addition salt may be converted into another by neutralizing one salt, for example, the hydrochloride, resulting in the free base, and then reacidifying with a different selected mineral or organic acid, to prepare another pharmaceutically-acceptable acid addition salt, as is conventional in the art. As will be recognized by a person of ordinary skill in the art, pharmaceutically acceptable salts of an active agent are commonly used. Therefore, for the sake of brevity, the compounds of the present invention may or may not specifically recite pharmaceutically acceptable salts thereof, yet, such salts are included in the reference to the active agent or compound.

A variety of measures to evaluate the effect of memantine and/or phenserine on the cognitive ability of a subject are known in the art. For example, the Severe Impairment Battery (SIB) to assess attention, orientation, language, memory, and social interactions, the modified AD Cooperative Study - Activities of Daily Living (ADCS-ADL) scale, which assesses the ability of subjects to eat, dress, bathe, travel, shop and perform household chores, the Behavioral Rating Scale for Geriatric subjects (BGP), which assesses day-to-day functioning, and the Clinical Global Impression of Change (CGI-C), which assesses the overall condition of the subjects, may be used. The measure of cognitive ability may be used to monitor the progression of the disease, relative to untreated subjects.

The cognitive effect may be assayed using a T-maze (Patel *et al*., (1998) Phenserine, a Novel Acetylcholinesterase Inhibitor, Attenuates Impaired Learning of Rats in a 14-unit T-maze Induced by Blockade of the N-methyl-D-aspartate receptor, *NeuroReport* 9(1):171-176). In addition, Pavlovian fear conditioning may be used to assay cognitive function. For example, mice may be condition by receiving 3 to 5 tone-foot shock trials in a conditioning chamber *(see,* Maren, S. (1999) Neurotoxic Basolateral Amygdala Lesions Impair Learning and Memory But Not the Performance of Conditional Fear in *Rats, J. Neurosci.* 19(19):8696-8703).

For total Aβ levels, the rabbit polyclonal antibody no. 3160 (1-40 residues of Aβ) is used as a capture antibody for all species of Aβ (Aβ1-40 and Aβ1-42), whereas mAb 4G8 (17-25 residues of Aβ) is used to detect Aβ levels, and the values are expressed as the mean of independent assays *(see,* Suzuki, N., *et al*. (1994) Science 264:1336-1340).

Total Aβ and Aβ42 levels may also be assayed in guanidine lysates as described (Johnson-Wood, *et al*. (1997) Proc. Natl. Acad. Sci. USA 94:1550-1555.). In brief, tissue, e.g., hippocampal or cortical, is homogenized in a denaturing buffer containing 5 M guanidine plus protease inhibitors. The extracts are diluted and analyzed in denaturing ELISAs containing a final concentration of 500 mM guanidine for total Aβ or Aβ42.

To evaluate the potential soluble pools of brain Aβ, a carbonate extraction may be performed (100 mM carbonate/50 mM NaCl/protease inhibitors, pH 11.5), for example, on hippocampal and cortical tissue (1:20, wt/vol) on ice. Tissue samples are Dounce homogenized and spun in a microcentrifuge at 14,000 rpm for 15 min at 4°C. The supernatant is placed in a fresh tube on ice and the pH of the lysate is neutralized to 7.4 with 1 M Tris (pH 6.8). The carbonate soluble pool of total Aβ is determined with denaturing (guanidine) and nondenaturing (lacking guanidine) ELISAs. An additional Aβ ELISA may be used to identify possible oligomeric species of Aβ. A monoclonal antibody directed against the first five residues of Aβ is used for both capturing and detecting Aβ.

Co-administration of a HMG CoA reductase inhibitor, such as lovastatin or pravastatin, and an AβI, such as phenserine, may provide an improved treatment for cognitive disorders. For example, the dosage of phenserine may be effectively limited by the response of a subject's cholinergic system. Over stimulation of the cholinergic system, which may result from high doses of a phenserine compound having AChEI activity, can produce trembling and other undesirable side effects. The method of the invention allows the dose of a phenserine compound having significant AChEI activity to be adjusted to provide a desirable level of cholinergic treatment. Furthermore, administration of phenserine has been shown to reduce production of β amyloid precursor protein (β-APP), thereby decreasing Aβ accumulation in a subject. This cholinergic and β-APP effect is augmented by co-administration of a HMG CoA reductase inhibitor, for example, lovastatin or pravastatin, which is believed to provide an improved treatment for AD. Augmentation of the activity of a phenserine compound by a HMG-CoA reductase includes synergistic effects, wherein the dosage of one or more compounds may be significantly reduced when used in combination with another compound.

The reported effects of HMG CoA reductase inhibitors, such as simvastatin and atorvastatin, on Aβ production have yielded an indefinite answer. At least one group has reported that statins have no effect on Aβ production (*see*, for example, Wiklund *et al*. (2004) Plasma Levels of Beta-amyloid (1-40), Beta-amyloid (1-42), and Total Beta-amyloid Remain Unaffected in Adult Patients with Hypercholesterolemia After Treatment with Statins, *Arch. Neurol*. 61(3):333-337). In contrast, other groups have reported that statins do decrease Aβ production *(see,* for example, Fassbender *et al*. (2001) Simvastatin Strongly Reduces Alzheimer's Disease Ab42 and Ab40 Levels *in vitro* and *in vivo*, *Proc. Natl. Acad. Sci. USA* 98(10):5856-61; and Simons *et al*. (2002) Treatment with Simvastatin in Normocholesterolemic Patients with Alzheimer's Disease: A 26-week Randomized, Placebo-controlled, Double-blind Trial, *Ann. Neurol*. 52(3):346-350). Therefore, the literature is unclear as to the effect of a statin on Aβ production. Co-administration of a phenserine compound and a HMG CoA reductase inhibitor is believed to provide an improved treatment for cognitive disorders, such as Alzheimer's disease, by lowering Aβ levels, for example, by synergistically lowering Aβ levels and/or reducing inflammation.

In accordance with this invention, a phenserine compound may comprise more than one phenserine compound. For example, if the phenserine compound is phenserine, the invention includes addition of (3aR)-1,3a,8-trimethyl-1,2,3,3a,8,8a-hexahydropyrrolo[2,3-b]indol-5-yl phenylcarbamate, a (+) isomer of phenserine, and its pharmaceutically acceptable salts. Examples of cognitive disorders that may be treated, prevented or the progression delayed, include those that are characterized by increased Aβ levels. (+)-Penserine, the non-natural (+) isomer of phenserine, has minimal anticholinesterase activity, therefore, the Aβ decreasing activity of (+) 9 -N-phenylcarbinol esroline may be utilized in addition to an AChEI activity possessed by (-)-phenserine, without over stimulating the cholinergic system of a subject.

Use of a phenserine compound and a HMG-CoA reductase inhibitor to delay progression or onset of a cognitive disorder associated with increased levels of Aβ is believed to provide an effective method of delaying the onset or progression of the cognitive disorder by reducing the production of Aβ and reducing inflammation in the subject, thereby providing an improved method of treatment.

The invention is further explained with the aid of the following illustrative Examples.

### EXAMPLE I

To evaluate the role of a statin and/or phenserine in AD pathology, PDAPP homozygous mice are used to assess the effect of the drugs. Control animals receiving neither drug are evaluated against animals receiving one or both drugs. For example, lovastatin is administered at a dosage of 5 or 500 mg/kg/day and/or phenserine is administered at a dosage of 5 or 10 mg/kg *bid*.

Four treatment groups are established having at least 6 animals per group, wherein the animals are old (12-14 month) PDAPP mice homozygous (+/+) for the APP V717F transgene, a transgenic mouse model that develops AD-like neuropathology. Treatment group 1 is a control group receiving vehicle only, group 2 receives lovastatin at either 5 or 500 mg/kg/day, group 3 receives phenserine at 2-5 mg/kg *bid,* and group 4 receives both Lovastatin and phenserine. Animals are treated for 4 weeks, with cognitive ability tested prior to treatment and at appropriate times throughout the treatment period. Aβ levels, for example, in the brain, are measured at the conclusion of the treatment period and assessed relative to the control group.

Animals receiving both phenserine and lovastatin are found to have improved cognitive function and to show reduced Aβ levels, for example, Aβ levels are reduced relative to the control group.

### EXAMPLE II

Capsules containing 10 mg of lovastatin and 10 mg of phenserine are made by incorporating pharmaceutically acceptable salts of lavastatin and phenserine into a gelatin capsule.

### EXAMPLE III

The major neuropathological characteristics of Alzheimer's disease (AD) are cerebrovascular amyloid depositions, tau phosphorylation, cholinergic deficits and oxidative stress (Lahiri et al, 2002; Luth et al, 2002; Selkoe and Shenk, 2003). Current research suggests that the neuronal cell death observed in AD may be attributed to apoptosis promoted by the amyloid beta peptide (Aβ), which is derived from β-amyloid precursor protein (APP). For example, familial cases of AD are caused by mutations in APP and presenilin-1 (PS1) genes. It has been shown that PS1 mutations perturb neuronal calcium homeostasis, increase Aβ production, and enhance the vulnerability of neurons to synaptic dysfunction, excitotoxicity, and apoptosis (Lee et al, 2002).

Notably, astrocytes with elevated levels of NOS have been observed in association with Aβ-deposits in AD and in APP transgenic (Tg) mice (Luth et al, 2001). However it is currently unknown whether Aβ generation directly induces apoptotic cell death or triggers an alternative pathway, NOS, that then leads to neurodegeneration. Moreover, there are reports indicating that statins may function through a mechanism involving NO (Jick *et al*. (2000) Statins and the Risk of Dementia, *Lancet* 356(9242): 1627-1631).

To determine if aberrant expression of NOS isoforms is a primary event in the pathogenic cascade of AD (and might thus be a potential therapeutic target), or if it reflects a secondary effect that occurs at more advanced stages of the disease process the following experiments were conducted by Lahiri *et al*. (2004) (-)-Phenserine's Action on Nitric Oxide Synthase Expression in Normal and APP/PS 1 Double Transgenic Mice, 8th annual Montreal/Springfield Symposium on Alzheimer - Continuing Medical Education Conference, April 14-17, 2004.

To determine whether or not excessive Aβ deposition would alter NOS activity, and thereby affect NOS-mediated superoxide formation leading to neurodegeneration, we compared both neuronal (n) NOS and inducible (i) NOS activity in brain extracts from Tg mice (expressing human APP with the Swedish double mutation plus PS-1) and nTg mice. In addition, the effect of Phenserine, a highly selective acetylcholinesterase inhibitor that lowers Aβ levels, was tested on NOS activity *in vivo*.

NOS activity was assayed by three paradigms: i) enzymatic activity of NOS by measuring the rate of conversion of ³H-arginine to 3H-citrulline (Bondy et al, 2002), ii) levels of neuronal NOS (nNOS) and iii) levels of inducible NOS (iNOS) by Western blotting with specific antibodies against nNOS and iNOS (Santa Cruz Biotechnology, Santa Cruz, CA). Proteins were analyzed by SDS-PAGE followed by Western immunoblotting and densitometry (Lahiri et al, 1998). All were studied in brain extracts from both APP/ PS-1 double transgenic and nTg mice of similar age and gender (5 month old males). The brain of Tg mice showed a robust level of Aβ deposition and associated neuropathology compared to those from nTg mice (Borchelt et al, 1997). Five brain regions (cortex, hippocampus, cerebellum, striatum and the remaining brain) from both Tg and nTg mice were homogenized and assayed for NOS enzymatic activity within the linear range of its detection. All brain extracts showed quantifiable levels of NOS activity, with Cerebellum extracts from the nTg mice showing the highest level of NOS activity, which was 4-fold higher than cortical extracts. Like the nTg brain, Tg cerebellum extracts showed the highest level of NOS activity. However, there was no significant difference in NOS activity between nTg and Tg brain extracts.

Furthermore, in Tg mice administered (-)-phenserine (2.5 mg/kg, i.p.), which lowered brain Aβ levels (by approximately 50%), no significant difference in NOS activity was found between treated and control Tg groups, though levels of nNOS protein were increased.

These results suggest that excess levels of Aβ failed to trigger a change NOS levels. Thus, it is believed that Aβ deposition does not contribute to a NOS-mediated nitric oxide release and subsequent generation of superoxide radicals. Similarly, treatment with an Aβ lowering cholinesterase inhibitor has no effect on NOS activity. Hence, whereas NOS isoforms may be part of AD pathology, they are probably secondary to the amyloid pathology; a view consistent with previous reports (Heneka et al., 2001; Luth et al., 2001).

These results suggest that excess levels of Aβ failed to trigger NOS activity or change NOS levels. Therefore, it is believed that NOS is an unlikely mechanism of action for the effect of statins on AD.

### EXAMPLE IV

Phenserine is administered to subjects diagnosed with mild to moderate AD for a six-month treatment period in a randomized placebo-controlled double-blind clinical trial. Additional medications administered to the subjects are recorded. The subjects in the trial, or the data from the trial, are sorted based on whether the subject was also administered a HMG-CoA reductase inhibitor. The effect of HMG-CoA reductase inhibitor with and without phenserine is analyzed to demonstrate the improved treatment or delayed progression of AD in subjects co-administered a HMG-CoA reductase inhibitor and a phenserine compound.

Phenserine is administered at 10 mg twice daily or 15mg twice daily. Control subjects receive a placebo. Patients undergo testing using standard memory and cognition tests that are the efficacy endpoints required by the US FDA and European regulatory authorities for potential marketing approval. Optionally, β-APP or Aβ levels are measured in the subjects.

Where sufficient subjects receive a particular HMG-CoA reductase inhibitor, the data generated from these subjects is analyzed separately to determine the combinatorial effect of phenserine and a particular HMG-CoA reductase inhibitor.

It is found that co-administration of a HMG-CoA reductase inhibitor and phenserine provides an improved treatment method. Co-administration of an HMG-CoA, *e,g,*, lovastatin, and phenserine are found to synergistically delay progression of AD.

## Claims

1. Use of a first compound comprising an effective amount of phenserine and an effective amount of a second compound comprising a HMG CoA reductase inhibitor in the manufacture of a medicament for delaying progression of Alzheimer's Disease.

2. The use according to claim 1, wherein said HMG CoA reductase inhibitor is selected from the group consisting of atorvastatin, rivastatin, mevastatin, lovastatin, pravastatin, velostatin, fluvastatin, and combinations thereof.

3. The use according to claim 1, wherein said second compound is simvastatin.

4. The use according to claim 1, claim 2 or claim 3, wherein the first compound is formulated to deliver about 30 mg/day.

5. The use according to claim 1, claim 2 or claim 3, wherein the first compound is formulated to deliver about 40 mg/day.

6. The use according to any one of claims 1-5, wherein the delay in the progression of Alzheimer's Disease occurs over the course of at least a year.

7. The use according to any one of claims 1-6, wherein the first compound is phenserine.

8. The use according to any one of claims 1-7, further comprising use of an effective amount of a third compound in the manufacture of a medicament a third compound to the subject.

9. The use according to claim 8, wherein the third compound is (+) 9 -N-phenylcarbinol esroline.

10. The use according to claim 9, wherein the (+) 9 -N- phenylcarbinol esroline is (+) 9 -N- phenylcarbinol esroline tartrate.

11. The use according to any one of claims 1-10, wherein the phenserine compound is (+)-phenserine.

12. The use according to any one of claims 1-10, wherein the phenserine compound is N¹,N⁸-bisnorcymserine.

13. The use according to any one of claims 1-10, wherein the phenserine compound is (-)-N¹,N⁸-bisnorcymserine.

14. The use according to any one of claims 1-10, wherein the phenserine compound is tolserine.

15. The use according to any one of claims 1-10, wherein the phenserine compound is (-)-tolserine.

16. The use according to any one of claims 1-15, wherein the effective amount of a second compound comprising a HMG CoA reductase inhibitor is sufficient to reduce inflammation.

17. A composition comprising an effective amount of a phenserine compound or a pharmaceutically acceptable salt or ester thereof and an effective amount of a HMG CoA reductase inhibitor or pharmaceutically acceptable salt or ester thereof, wherein said composition is useful in the treatment or prevention of a cognitive disorder.

18. The composition of claim 17, comprising one or more pharmaceutically-acceptable diluents, excipients, or carriers.

19. The composition of any one of claim 17 or claim 18, wherein said effective amount of the phenserine compound is between about 10 mg and about 30 mg.

20. The composition of claims 17-19, wherein said effective amount of the phenserine compound is 15 mg.

21. The composition of any one of claims 17-20, wherein said cognitive disorder is Alzheimer's disease.

22. The composition of any one of claims 17-21, wherein the pharmaceutically acceptable salt of phenserine comprises phenserine tartrate.

23. A kit for treatment or prevention of a cognitive disorder, the kit comprising a first dosage unit comprising an effective amount of a phenserine compound or a pharmaceutically acceptable salt or ester thereof and a second dosage unit comprising an effective amount of a HMG CoA reductase inhibitor or pharmaceutically acceptable salt or ester thereof.

24. The kit of claim 23, wherein the phenserine compound is selected from the group consisting of (-)-phenserine, (+)-phenserine, (-)-N¹,N⁸-bisnorcymserine, (+)-N¹,N⁸-bisnorcymserine, (-)-tolserine, (+)-tolserine and pharmaceutically acceptable salts thereof.
